# EUROPEAN PATENT APPLICATION

(11) **EP 0 860 157 A1**
(43) Date of publication of application: **26.08.1998**
(21) Application number: 98200345.1
(22) Date of filing: 05.02.1998
(51) Int. Cl.: A61F 11/00, A61M 13/00

(54) **Device for treating the human ear**

(30) Priority: 10.02.1997 NL 1005243
(71) Applicant: Entermed B.V., 3440 AE Woerden (NL)
(72) Inventor: Versluis, Teunis Huibert, 2941 RJ Lekkerkerk (NL)
(74) Representative: de Bruijn, Leendert C.

(57) **Abstract**

A device for treating (parts of) the interior of the human ear, in particular the radical cavity formed when the organ of hearing is removed, comprises a guide member, such as an ear funnel, which can be arranged in the auditory duct and has a passage via which the interior of the ear is accessible, heating means for heating air, feed means for feeding the heated air into the interior of the ear, as well as control means for controlling the heating means in such a manner that the air delivered into the interior of the ear is approximately at body temperature.

## Description

The invention relates to the field of carrying out treatments in the interior of the human ear. In particular, it concerns treatments which take place in people who have had the interior of their ear removed to a greater or lesser extent for certain reasons, for example a chronic infection. As a result of an intervention of this nature, there is a so-called radical cavity in the interior of the ear, which cavity adjoins the organ of balance.

It is easy for ear wax and skin cells which have been shed to build up in this cavity, with the result that the risk of infection in the cavity increases. Therefore, a radical cavity of this kind has to be inspected and cleaned from time to time, mostly by means of suction, by an ear, nose and throat specialist. When doing this, the physician uses an ear funnel which is introduced into the auditory duct. By manipulating the ear funnel in a suitable manner, the physician can gain access to the radical cavity. He can then carry out the necessary treatment via the passage in the ear funnel.

This treatment is carried out using various instruments. In view of the need to remove dirt, a suction tube is frequently introduced, which can generate such a strong vacuum that the dirt is sucked out, if necessary after having been dislodged by manipulation of the said suction tube. The suction is accompanied by absolute pressures which may fall to 0.12 bar, at a flow rate of 40 l/min.

A disadvantageous effect of these treatments is that cooler ambient air at ± 20°C flows into the interior of the ear to replace the sucked-out air which was originally present in the ear and was at ± 37°C. There is therefore a temperature drop of ± 17°C within a short period of time in the cavity. This causes considerable irritation to the horizontal duct of the organ of balance, which is exposed in the radical cavity. The result is symptoms of dizziness, problems of balance and sometimes nausea and vomiting. These reactions are extremely unpleasant for the patient. For the ear, nose and throat specialist, these reactions mean that his possible actions are limited. These reactions often mean that the necessary suction has to be interrupted prematurely.

The object of the invention is to provide a treatment device of this kind which causes these problems to a considerably lesser extent, or does not do so at all. This object is achieved by means of a device for treating the interior of the human ear, in particular the radical cavity which is caused by an operation, which device comprises a guide member, such as an ear funnel, which can be arranged in the auditory duct and has a passage via which the interior of the ear is accessible, heating means for heating air, supply means for supplying the heated air into the interior of the ear, as well as control means for controlling the heating means in such a manner that the air delivered into the interior of the ear is approximately at body temperature.

The device according to the invention ensures that the air which is to be removed by suction is directly replaced with air which is approximately at body temperature. This avoids the formation of convection currents in the organ of balance, with the result that the person in question remains in a better condition and the treatment can be carried out without interruption.

The space available in the interior of the ear is very limited, particularly if the guide member, for example an ear funnel, has already been introduced into the ear. Heated air is therefore preferably supplied by means of a guide member, into the passage of which a short length of tubing opens out, which tubing forms part of the air-supply means. The tubing is situated in the region of the outer opening of the guide member, so that the latter can still always be positioned in the auditory duct without problems.

The guide member is usually funnel-shaped or trumpet-shaped; the tubing preferably runs transversely to the axis of the said guide member. In connection with the desired flow of the heated air into the guide member, the tubing is connected to the funnel-shaped or trumpet-shaped guide member with a curve towards the wall thereof. The guide member serves to close off the entry to the auditory duct completely, so that it is impossible for any unheated air to leak inwards.

The tubing is clamped in a rubber sealing ring at the end of the handle, for the purpose of manipulation of the guide member. Furthermore, a flexible air-supply hose, which is connected to the tubing, is attached to the handle.

An electrical heating source is also provided for heating the supplied air, as well as a pump for supplying the air. A sensor for measuring the air temperature may be situated between the heating source and the tubing.

Since the heated air has to reach the interior of the ear within rather accurately defined temperature limits, it is preferred to accommodate the heating source and the sensor in the handle and to connect the heating source, via the air hose, to a pump which is present in a housing. The air, which has to be heated to a temperature which is a few degrees too high, can then not cool down excessively.

The power supply cable for the heating source, as well as the sensor cable, can run through the inside of the supply hose, leaving free space for the air to flow. An advantage of this design is that the hose cannot be pressed flat, thus ensuring protection against unintentional interruption of the supply of heated air.

The housing may have selection means for selecting a desired temperature, as well as a control member, which is connected to the selection means, for controlling the heating source on the basis of the temperature level set as well as the signals emanating from the sensor.

The invention also relates to the guide member for use in the device described above.

The invention will now be explained in more detail with reference to an exemplary embodiment illustrated in the figures, in which:
Figure 1 shows a diagrammatic depiction of the device according to the invention.
Figure 2 shows an enlarged detail, partially in section, of the device in accordance with Figure 1.

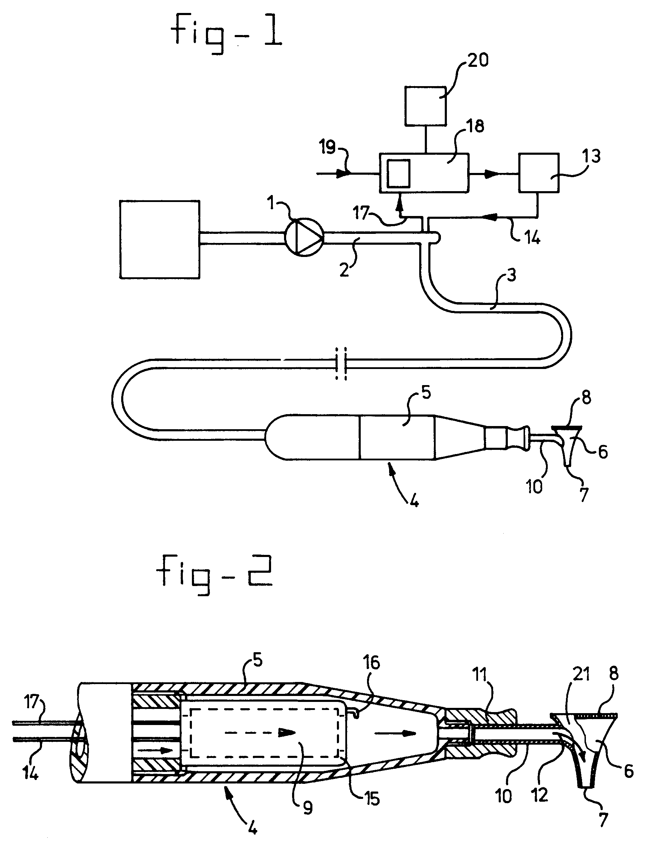

The device illustrated in Figure 1 comprises an electrically driven air pump 1, which is connected via a line 2 to the flexible hose 3. This flexible hose 3 is connected to the instrument 4, which comprises a handle 5 as well as a guide member 6 according to the invention. The instrument 4 can be held in the hand of an ear, nose and throat specialist, the ear funnel 6 being manoeuvred with the narrow end 7 in the auditory duct of the ear. Via the wide entry 8 and the passage 21, the physician can then use yet another instrument, for example a suction tube, to gain access to the interior of the ear with a view to cleaning it.

When sucking the ear clean by means of a suction tube, cooler ambient air normally flows inwards via the ear funnel 6. This is unpleasant for the patient, since the cooler air causes the liquid in the organ of balance to begin to circulate, resulting in problems of balance and a feeling of dizziness and nausea.

According to the invention, the pump 1 can now introduce air at the desired temperature into the ear funnel 6 by means of the tubing 10, via line 2, the hose 3 and past the heating device 9 present in the instrument 4, after which this air can flow into the interior of the ear. The tubing 10 is attached to the end of the handle 5 by means of a rubber sealing sleeve 11.

Via a curved section 12, the tubing 10 merges into the trumpet-shaped, narrowing wall of the ear funnel 6, thus ensuring that the ambient air which could enter via the wide end 8 of the ear funnel 6 can also flow inwards.

Since the heating member 9 is situated in the handle, i.e. the air is brought to the correct temperature just upstream of the interior of the ear, it is ensured that the air cannot cool down excessively. Preferably, the air delivered through the heating member 9 is kept at a temperature which is slightly higher than body temperature, for example in the range from 38°C to 42°C.

The heating member 9 is controlled by means of the electrical power supply 13, which is connected by means of line 14 to the electrical heating member 9. In order to maintain the desired temperature, a sensor 16, which is connected to the control device 18 via line 17, is placed in the region of the outlet opening 15 of the heating member. The control device 18 is supplied with mains voltage 19. The desired temperatures can be set by means of selection device 20, for example in the range from 38°C to 42°C. Depending on this setting and on the signals emanating from the sensor 16 via line 17, the control device actuates transformer 13, which then regulates the electrical heating device 9 using a voltage of 24 volts via line 14.

In Figure 2, arrows indicate the air flow direction of the air which is supplied via the hose 3 and is heated in the electrical heating device 9.

It should be noted that the hose 3 consists of flexible material, in order to facilitate manoeuvring with the instrument 4. Owing to the presence of the lines 14 and 17 in the hose 3, the latter is protected against being closed off completely as a result of the hose being crushed.

## Claims

1. Device for treating (parts of) the interior of the human ear, in particular the radical cavity formed when the organ of hearing is removed, which device comprises a guide member (6), such as an ear funnel, which can be arranged in the auditory duct and has a passage (21) via which the interior of the ear is accessible, heating means (9) for heating air, supply means (1, 2, 3, 10, 12, 21) for supplying the heated air into the interior of the ear, as well as control means (13, 14, 16, 18, 20) for controlling the heating means (9) in such a manner that the air delivered into the interior of the ear is approximately at body temperature.

2. Device according to Claim 1, in which a short length of tubing (10) opens into the passage (21) in the guide member (6), which tubing (10) forms part of the air-supply means (3).

3. Device according to Claim 2, in which the guide member (6) is funnel-shaped or trumpet-shaped, and the tubing (10) runs transversely to the axis of the guide member (6).

4. Device according to Claim 3, in which the tubing (10) is connected to the funnel-shaped or trumpet-shaped guide member (6) with a curve (12) towards the wall thereof.

5. Device according to one of Claims 2-4, in which a handle (5) is attached to the tubing (10), for the purpose of manipulation of the guide member (6).

6. Device according to Claim 5, in which a flexible air-supply hose (3), which is connected to the tubing (10), is attached to the handle (5).

7. Device according to Claim 6, in which an electrical heating source (9) is provided for heating the supplied air, as well as a pump (1) for supplying the air.

8. Device according to Claim 7, in which a sensor (16) for measuring the air temperature is situated between the heating source (9) and the tubing (10).

9. Device according to Claim 8, in which the heating source (9) and the sensor (16) are situated in the handle (5), and the heating source (9) is connected, via the air hose (13), to a pump (1) which is present in a housing.

10. Device according to Claim 9, in which the power supply cable (14) for the heating source (9), as well as the sensor cable (17), run through the inside of the supply hose (3), leaving free space for the air to flow.

11. Device according to Claim 9 or 10, in which the housing has selection means (20) for selecting a desired temperature, as well as a control member (18), which is connected to the selection means (20), for controlling the heating source (9) on the basis of the temperature level set as well as the signals emanating from the sensor (16).

12. Guide member (6) for a device according to one of the preceding claims for treating the interior of (parts of) the human ear, in particular the radical cavity which is formed when the organ of hearing is removed, which guide member (6) can be introduced into the auditory duct of the ear and has a passage (21), via which the interior of the ear is accessible, into which passage (21) in the guide member (6) a short length of tubing (10) opens out, which tubing can be connected to air-supply means (3).

13. Guide member according to Claim 12, in which the guide member (6) is funnel-shaped or trumpet-shaped, and the tubing (10) runs transversely to the axis of the guide member (6).

14. Guide member according to Claim 13, in which the tubing (10) is connected to the funnel-shaped or trumpet-shaped guide member (6) with a curve (12) towards the wall thereof.
